# EUROPEAN PATENT SPECIFICATION

(11) **EP 4 255 382 B1**
(45) Date of publication and mention of the grant of the patent: **26.02.2025**
(21) Application number: 21819885.1
(22) Date of filing: 03.12.2021
(51) Int. Cl.: A61K 8/88, A61Q 15/00

(54) **ANTIPERSPIRANT COMPOSITIONS**
SCHWEISSHEMMENDE ZUSAMMENSETZUNGEN
COMPOSITIONS ANTITRANSPIRANTES

(30) Priority: 07.12.2020 EP 20212236
(43) Date of publication of application: 11.10.2023
(73) Proprietor: Unilever IP Holdings B.V., 3013 AL Rotterdam (NL); Unilever Global IP Limited, Wirral, Merseyside CH62 4ZD (GB)
(72) Inventor: SKINNER, Richard, Bebington Wirral Merseyside CH63 3JW (GB); TAYLOR, Cheryl Anne, Bebington Wirral Merseyside CH63 3JW (GB)
(74) Representative: Whaley, Christopher
(86) International application number: PCT/EP2021/084121
(87) International publication number: WO 2022/122574

(56) References cited:
- EP-B1- 2 717 965
- WO-A1-2014/204439
- WO-A2-2009/150090
- JP-A- S62 286 918
- US-A1- 2017 157 017

## Description

### Field of Invention

The present invention is in the field of antiperspirant compositions and methods of achieving reduced perspiration.

### Background

There are numerous references to the use of polypeptides comprising lysine in cosmetic compositions in the prior art, but relatively few relate to antiperspirant compositions.

DE 10 2014 222 023 A1 (Henkel, 2016) discloses aluminium free antiperspirant compositions comprising a gamma-aminobutyric acid or similar amino acid and a broadly defined "design protein".

WO 2012/168360 A2 (BDF, 2012) discloses active substance combinations comprising epsilon-polylysine, piroctone olamine and/or climbazole.

US 2017/157017 (Henkel, 2017) discloses antiperspirant compositions comprising a design protein and which are exempt of aluminium and/or zirconium antiperspirant actives. Preferred design proteins are selected from polylysine, polyarginine, polyglutamine, polyhistidine, polyproline, as well as mixtures thereof.

DE 102014216893 A1 (Henkel, 2016) discloses the use of at least one specific protein to reduce the amount of antiperspirant compound needed in an antiperspirant composition.

JP2004035461 A2 (Chisso Corp., 2004) discloses deodorant compositions comprising epsilon-polylysine which operates as an antibacterial agent and thereby reduces malodour.

### Summary of Invention

It is an object of the invention to reduce perspiration on the surface of the human body, in particular by the use of selected antiperspirant agents that do not comprise aluminium or zirconium or any other metal salts.

It is a further object of the invention to reduce perspiration on the surface of the human body by the use an antiperspirant composition comprising an organic antiperspirant active.

The invention aims to provide the non-therapeutic cosmetic use of a polypeptide comprising multiple alpha-linked lysine residues as an antiperspirant agent, wherein the polypeptide is an alpha-linked homopolypeptide of lysine having a molecular weight of from 15,000 to 30,000.

In a second aspect of the invention, there is provided a cosmetic method of reducing perspiration comprising the topical application of a composition comprising a polypeptide comprising multiple alpha-linked lysine residues as an antiperspirant agent and a cosmetically acceptable carrier fluid as defined in claims.

In a third aspect of the invention, there is provided an antiperspirant composition comprising a polypeptide comprising multiple alpha-linked lysine residues; a cosmetically acceptable carrier fluid; and a preservative and/or deodorant active as defined in claims.

In a fourth aspect of the invention, there is provided an antiperspirant product comprising a composition as described in the third aspect of the invention and a dispenser for said composition, the dispenser comprising containment means and application means for the composition.

In the cosmetic method described in the second aspect of the invention, the composition preferably comprises a cosmetically acceptable carrier fluid and a preservative and/or deodorant active.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, all percentages, ratios and amounts are by weight, unless otherwise indicated.

Herein, all percentages, ratios and amounts are to be understood to be modified by the word "about" where appropriate.

Herein, the word "comprising" is intended to mean "including" but not necessarily "consisting of", i.e., it is non-exhaustive.

Herein, cosmetic methods are to be understood as a non-therapeutic in nature and *vice versa.*

Herein, references to an amount of a compound or an active refer to the total amount of compounds or actives of the type indicated.

Herein, "application" and "applied" relate to application to the surface of the human body, in particular the underarm regions, unless the context dictates otherwise.

Herein, the term "polypeptide" refers to any polypeptide comprising two or more amino acid residues and therefore covers polypeptides sometimes referred to as peptides or oligopeptides.

Herein, molecular weights for polypeptides refer to number average molecular weights.

The invention typically involves the topical application of the antiperspirant active to the underarm regions of the human body, otherwise known as the axillae.

A polypeptide comprising multiple alpha-linked lysine residues is an essential feature of the present invention. Lysine is an amino acid having two amino groups, located alpha and epsilon to the carboxylic acid of the amino acid:

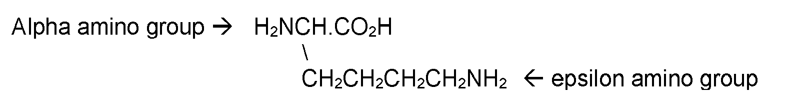

Alpha-linked lysine residues are linked to the next amino acid by an amide bond between the amino group alpha to the carboxylic acid group of a first lysine residue and a carboxylic acid group of a second amino acid, whether lysine or another amino acid.

The polypeptide preferably comprises at least three alpha-linked lysine residues, more preferably at least four alpha-linked lysine residues and most preferably at least five alpha-linked lysine residues.

In particularly preferred embodiments, the lysine residues in the polypeptide as described in the paragraph immediately above are consecutive, i.e. the polypeptide preferably comprises an alpha-linked trilysine residue, more preferably an alpha-linked tetralysine residue and more preferably an alpha-linked pentalysine residue.

The polypeptide is a homopolypeptide of lysine.

The polypeptide is an alpha-linked homopolypeptide of lysine having a molecular weight of from 15,000 to 30,000.

In each of the embodiments and preferences thereon described herein, the lysine residues in the polypeptide preferably each have their natural, L-lysine, stereochemistry.

The antiperspirant active is preferably used from a composition comprising it at a level of from 0.05 to 20%, preferably from 0.2 to 15% and more preferably from 0.5 to 10%, excluding any volatile propellant therein.

In preferred embodiments, the polypeptide is delivered to the skin surface at a concentration of at least 0.05% by weight and more preferably at a concentration of from 0.05 to 1.0% by weight.

Compositions according to the invention comprise a carrier fluid that aids the delivery of the polypeptide comprising multiple alpha-linked lysine residues to where it is required on the skin surface, which is typically in the vicinity of the sweat pores, particularly in the underarm areas of the human body.

The carrier fluid used in conjunction with the present invention must be cosmetically acceptable and it generally aids in the delivery of the non-pore blocking antiperspirant agent to the surface of the human body. The non-pore blocking antiperspirant agent is typically suspended or dissolved in the carrier fluid.

The carrier fluid may comprise from 10 to 99% of the composition, excluding any volatile propellant that may be present therein.

In preferred aspects of the present invention, the carrier fluid for the non-pore blocking antiperspirant agent comprises a C₂-C₆ vicinal diol.

Herein, C₂-C₆ vicinal diols are compounds with from 2 to 6 carbon atoms in which two neighbouring carbon atoms bear hydroxyl groups.

Examples of C₂-C₆ vicinal diols include ethylene glycol, propylene glycol, glycerol and hexane-1,2-diol.

Preferred C₂-C₆ vicinal diols exclude compounds comprising more than 3 hydroxyl groups.

Preferred C₂-C₆ vicinal diols are C₂-C₄ vicinal diol, i.e. compounds with from 2 to 4 carbon atoms in which two neighbouring carbon atoms bear hydroxyl groups.

When employed, the C₂-C₆ vicinal diol in compositions of the invention is preferably present at from 1 to 50%, more preferably from 2 to 40% and most preferably from 5 to 25%.

A preferred additional component of the carrier fluid is ethanol. This may comprise up to 80% of the total composition, but is typically restricted to 70%, and often less than 60%. When employed ethanol typically comprises at least 10%, preferably at least 20%, and more preferably at least 40% by weight of total composition. Each of these preferred minimum levels of ethanol may be limited by the aforementioned maximum levels of ethanol.

A preferred additional component of the carrier fluid is water, especially when employed in conjunction with ethanol, to give an aqueous ethanol carrier fluid. Water may comprise up to 90% of the total composition, but is typically restricted to less than 60%, and often less than 50%. When employed water typically comprises at least 10%, preferably at least 20%, and more preferably at least 30% by weight of total composition. Each of these preferred minimum levels of water may be limited by the aforementioned maximum levels of water.

A preservative or deodorant active is at least a preferred feature in each aspect of the invention.

Preservatives help to prevent microbial contamination of the composition. They are particularly important when the composition does not comprise a conventional, astringent aluminium containing antiperspirant salt, which is the case in preferred embodiments.

Deodorant actives help counter malodour development on the surface of the human body. They are particularly important when the composition does not comprise a conventional, astringent aluminium containing antiperspirant salt.

The preservative and/or deodorant active is preferably an organic anti-microbial agent. Such agents may be selected from any of those known in the art, provided reasonable skill is used to avoid any incompatibilities. An organic anti-microbial agent is a particularly preferred additional component when the composition used does not include a conventional, astringent aluminium containing antiperspirant salt.

The preservative and/or deodorant active is typically used at a level of from 0.01 to 5% by weight of the composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C. Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S. A. Makin and M. R. Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as tea tree oil and thyme oil, climbazole, octapyrox, ketoconazole, zinc pyrithione and mixtures thereof.

Compositions used in accordance with the present invention may optionally include conventional astringent aluminium-containing antiperspirant salts, although in preferred embodiments such salts are excluded.

Conventional astringent aluminium-containing antiperspirant salts include aluminium, zirconium and mixed aluminium/ zirconium salts, including both inorganic salts, salts with organic anions and complexes. Preferred astringent salts include aluminium, zirconium and aluminium/zirconium halides and halohydrate salts, such as chlorohydrates. Especially preferred aluminium halohydrate salts, known as activated aluminium chlorohydrates, are described in EP 6,739 (Unilever PLC and NV). Zirconium aluminium chlorohydrate actives are also preferred materials, as are the so-called ZAG (zirconium-aluminium-glycine) complexes, for example those disclosed in US 3,792,068 (Procter and Gamble Co.). Zinc phenol sulphonate may also be used, preferably at up to 3% by weight of the composition.

When employed, preferred levels of incorporation of conventional astringent aluminium-containing antiperspirant salts are from 0.5% to 60%, particularly from 5% to 40%, and especially from 5% or 10% to 30% or 35% of the composition.

In preferred embodiments of the invention, conventional astringent aluminium-containing antiperspirant salts are not employed.

A preferred optional component of compositions of the invention is a fragrance, typically at a level of from 0.1 to 5% of the total composition. In compositions also comprising water, the fragrance is preferably accompanied by a fragrance solubiliser, typically a nonionic surfactant used a concentration of from 0.1 to 5% of the total composition.

Thickening agents may be employed in compositions of the invention. Such agents increase the viscosity of or solidify the carrier fluid in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 to 40%. When a hydroxyalkyl cellulose thickening agent is employed, this is typically used at a level of from 0.2 to 10% by weight.

In some embodiments, the present invention may involve a stick or soft solid composition. In such compositions, a thickener is an essential additional component and is often described as a gelling agent or structurant.

The thickening agents used in stick compositions according to the invention are preferably selected from fibre-forming non-polymeric gelling agents and waxes, optionally supplemented by particulate silica and/or an oil-soluble polymeric thickening agent.

When employed, the thickening agent often comprises a wax. Thickening waxes typically melt at above 40°C and particularly at between 55 and 95°C. Waxes can include ester waxes, including C12 to C24 linear fatty alcohols, waxes obtained from animals or plants, often after hydrogenation, silicone elastomers and silicone waxes. The thickening agent can comprise a mixture of particulate thickening agents, a mixture of waxes or a mixture of both types of material.

Waxes employed herein as thickening agents are often selected from hydrocarbons, linear fatty alcohols, silicone polymers, esters of fatty acids or mixtures containing such compounds along with a minority (less than 50% w/w and often less than 20% w/w) of other compounds. Naturally occurring waxes are often mixtures of compounds which include a substantial proportion of fatty esters.

Examples of hydrocarbon waxes include paraffin wax, ozakerite, microcrystalline wax and polyethylene wax, the last named desirably having an average molecular weight of from 300 to 600 and advantageously from 350 to 525.

Examples of linear fatty alcohols include those containing from 14 to 40 carbon atoms and often from 16 to 24. Preferred thickening agents of this class are stearyl alcohol and behenyl alcohol, with stearyl alcohol being especially preferred.

Examples of ester waxes include esters of C₁₆-C₂₂ fatty acids with glycerol or ethylene glycol.

Examples of natural waxes include beeswax, wool wax and spermaceti wax of animal origin, and caster wax, jojoba wax, carnauba wax and candelilla wax which are of vegetable origin.

Further waxes employable herein are silicone polymer waxes.

Fibre-forming thickening agents are dissolved in the carrier oil at elevated temperature and on cooling precipitate out to form a network of very thin strands that thicken the carrier oil. Such fibre-forming thickeners include N-acyl amino-acid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

When employed, the thickening agent is typically used at a concentration of from 1.5 to 30%. When a fibre-forming thickening agent is employed, its concentration is typically in the range of from 1.5 to 15%. When a wax is employed, its concentration is usually selected in the range of from 10 to 30%, and particularly from 12 to 24% w/w.

In some embodiments, the present invention may involve an aerosol composition. In such compositions, a volatile propellant is preferred additional component. Preferred volatile propellants are liquefied gases, for example hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred.

The liquefied propellant gas is typically the major component of aerosol compositions used in conjunction with the invention, often comprising from 30 to 99% and preferably comprising from 50 to 95% of the total composition.

Other components that may be included in compositions according to the invention including those described in the following paragraphs.

Wash-off agents may be included, often in an amount of up to 10%, to assist in the removal of the formulation from skin or clothing. Such wash-off agents are typically nonionic surfactants such as esters or ethers containing a C₈ to C₂₂ alkyl moiety and a hydrophilic moiety comprising a polyoxyalkylene group (POE or POP).

Skin feel improvers (e.g. talc or finely divided high molecular weight polyethylene), may be included, typically in an amount from 1 up to 10%.

Skin moisturisers, such as glycerol or polyethylene glycol (e.g. mol. wt. 200 to 600) may be included, typically in an amount of up to 5%.

Skin benefit agents, such as allantoin or lipids, may be included, typically in an amount of up to 5%.

Dispensers suitable for use with the present invention comprise containment means and application means for the composition. They also typically comprise means for getting the composition from its containment means to its application means.

In embodiments where the composition is in a liquid form, the containment means is a reservoir for the composition and the application means is preferably a surface from which the composition may be applied to the skin.

In embodiments where the composition is in a solid or soft solid form, the containment means may be a stick barrel and the application means propels a composition comprising the polypeptide clear of the stick barrel and allows it to be rubbed onto skin surface.

In other embodiments, a composition comprising the polypeptide is sprayed onto the skin surface. In such embodiments, the containment means is typically a pressurised canister and the application means is a spray nozzle through which the composition is released under pressure on release of a valve.

### Examples

Herein, Examples according to the invention are designated by numbers and Comparative Examples are designated by letters.

The ACH disclosed in these examples is aluminium chlorohydrate, (Al Chlorhydrol 50, from Elementis). The active level indicated refers ACH anhydrous solids.

The polylysine disclosed in these examples is a homopolypeptide, alpha-linked, hydrochloride salt. MW 15,000-30,000 equating to 82-164 residues, unless otherwise indicated, as may be obtained from Sigma.

The samples detailed in Table 1 were prepared by the following method. For Comparative Example A and Examples 1 to 5, an aliquot of model sweat (137.5 µl) was added to an Eppendorf tube, followed by an aliquot of mucin (at 0.8%) in model sweat (312.5 µl). For Comparative Example A, an aliquot (50 µl) of ACH solution (at 3%) in water was also added. For examples 1 to 5, an aliquot (50 µl) of polylysine solution (at the concentration [in water] indicated in Table) was also added. For Comparative Example B (control sample), only the model sweat (187.5 µl) and mucin (at 8%) in model sweat ((312.5 µl) were added to the Eppendorf tube. The samples were prepared in triplicate and were each inverted twice to ensure good mixing.

After 2 hours at room temperature, the turbidity of the samples was measured. To do this, the contents of each tube was resuspended with a pipette and samples (100 µl) were placed in a 96 well microtitre plate. The absorbance of each plate at 590 nm was then measured using a plate-reader. The mean results for each sample are shown at the bottom of Table 1.

The figures given for ACH and polylysine refer to the concentration of the active in the aliquots added. The resulting concentration in the Eppendorf tube was one tenth of this.

The examples 6-9 are reference examples.

**Table 1**

| | Example | | | | | | |
|---|---|---|---|---|---|---|---|
| | A | 1 | 2 | 3 | 4 | 5 | B |
| Ingredient: | | | | | | | |
| ACH | 3 | -- | -- | -- | -- | -- | -- |
| Polylysine | -- | 2 | 1 | 0.5 | 0.1 | 0.05 | -- |
| Turbidity | 0.63 | 0.75 | 0.73 | 0.58 | 0.28 | 0.30 | 0.24 |

For each of the Examples tested, the resulting turbidity was greater than that of the control were only mucin was added to model sweat. The higher the value, the greater the potential for significant sweat reduction.

The samples detailed in Table 2 were prepared and tested by same method as for those in Table 1. Turbidity studies were conducted on these samples in triplicate, in the same manner as those detailed in Table 1. Double dose ("x2") involved adding 2x the volume of the ingredient solution into the same final volume of the total assay mixture as the 1x. The results of the turbidity studies are presented at the bottom of Table 2.

**Table 2**

| | **Example** | | | | | |
|---|---|---|---|---|---|---|
| | **A** | **6** | **7** | **8** | **9** | **B** |
| Dose: | x1 | x1 | x1 | x2 | x2 | -- |
| Ingredient: | | | | | | |
| ACH | 3 | -- | -- | -- | -- | -- |
| Pentalysine | -- | 3 | -- | 3 | -- | -- |
| Tetralysine | -- | -- | 3 | -- | 3 | -- |
| Turbidity | 0.60 | 0.29 | 0.27 | 0.33 | 0.27 | 0.24 |

The figures given for ACH and polylysine refer to the concentration of the active in the aliquots added. For the x1 dose samples, the resulting concentration in the Eppendorf tube was one tenth of this.

The pentalysine used was obtained from Sigma and had a peptide content of 55% or greater.

The tetralysine used was obtained from Sigma and had a peptide content of 50% or greater.

For each of the Examples tested, the resulting turbidity was greater than that of the control sample B, were only mucin was added to model sweat. This indicates potential for sweat reduction.

Selected compositions according to the invention are detailed in Tables 3 and 4. These may be prepared by methods known in the art.

**Table 3 - roll-on compositions**

| | **Example** | | | | |
|---|---|---|---|---|---|
| | **10** | **11** | **12** | **13** | **14** |
| Polylysine | 1.0 | 5.0 | 15.0 | 10.0 | 6.5 |
| Steareth-2 | 2.6 | 2.4 | 2.6 | -- | -- |
| Steareth-20 | 0.6 | 0.5 | 0.6 | -- | -- |
| Fragrance | 0.5 | 1.0 | 2.5 | 1.5 | 2.0 |
| Ethanol | -- | -- | -- | 40 | 60 |
| Cellulosic gum (thickener) | -- | -- | -- | 0.15 | 0.5 |
| Preservative | 0.2 | -- | 0.1 | -- | 0.2 |
| Water | To 100 | To 100 | To 100 | To 100 | To 100 |

The Steareth-2 and Steareth-20 served as thickener and fragrance solubiliser.

**Table 4 - cream and lotion compositions**

| | **Example** | | | | |
|---|---|---|---|---|---|
| | **15** | **16** | **17** | **18** | **19** |
| Polylysine | 5.0 | 5.0 | 15.0 | 10.0 | 6.5 |
| Self-emulsifying wax (Polawax GP200) (structurant) | 5.0 | -- | -- | 10.0 | -- |
| Ethoxylated fatty alcohol (emulsifier) | 4.5 | -- | 2.0 | 4.0 | -- |
| Propylene glycol | 3.0 | -- | -- | -- | -- |
| Preservative | 0.2 | -- | 0.2 | | 0.1 |
| Fragrance | 1.0 | 1.0 | 2.5 | 1.5 | 1.0 |
| Glycerol | -- | 1.0 | -- | 2.0 | -- |
| Isopropyl myristate | -- | -- | 1.7 | -- | -- |
| Ethanol | -- | 70.0 | 55.0 | -- | 75 |
| Deodorant active | -- | 0.2 | 0.3 | -- | -- |
| Water | To 100 | To 100 | To 100 | To 100 | To 100 |

## Claims

1. The non-therapeutic cosmetic use of a polypeptide comprising multiple alpha-linked lysine residues as an antiperspirant agent, wherein the polypeptide is an alpha-linked homopolypeptide of lysine having a molecular weight of from 15,000 to 30,000.

2. The use according to claim 1, wherein polypeptide is applied from a composition comprising a cosmetically acceptable carrier fluid and a preservative and/or deodorant active.

3. The use according to any one of the preceding claims, wherein the polypeptide is delivered to the skin surface at a concentration of at least 0.05% by weight.

4. An antiperspirant composition comprising a polypeptide comprising multiple alpha-linked lysine residues; a cosmetically acceptable carrier fluid; and a preservative and/or deodorant active, wherein the polypeptide is an alpha-linked homopolypeptide of lysine having a molecular weight of from 15,000 to 30,000.

5. A composition according to claim 4, wherein the polypeptide comprises from 0.05 to 10% of the composition, the carrier fluid comprises from 5 to 99% of the composition, and the preservative and/or deodorant active comprises from 0.01 to 5% of the composition, all percentages being by weight.

6. A composition according to any one of claims 4 to 5, comprising a fragrance.

7. A composition according to any of claims 4 to 6, wherein the carrier fluid comprises water.

8. A composition according to any of claims 4 to 7, wherein the carrier fluid comprises ethanol.

9. A product comprising a composition according to any of claims 4 to 8 and a dispenser for said composition, the dispenser comprising containment means and application means for the composition.

## Patentansprüche

1. Nicht-therapeutische kosmetische Verwendung eines Polypeptids, das mehrere alpha-verknüpfte Lysin-Reste umfasst, als schweißhemmendes Mittel, wobei das Polypeptid ein alpha-verknüpftes Homopolypeptid von Lysin mit einem Molekulargewicht von 15.000 bis 30.000 ist.

2. Verwendung nach Anspruch 1, wobei das Polypeptid aus einer Zusammensetzung aufgetragen wird, die eine kosmetisch akzeptable Trägerflüssigkeit und einen Konservierungs- und/oder Deodorantwirkstoff umfasst.

3. Verwendung nach irgendeinem der vorhergehenden Ansprüche, wobei das Polypeptid in einer Konzentration von mindestens 0,05 Gewichts-% an die Hautoberfläche abgegeben wird.

4. Schweißhemmende Zusammensetzung, die ein Polypeptid umfasst, umfassend mehrere alpha-verknüpfte Lysin-Reste, eine kosmetisch akzeptable Trägerflüssigkeit und einen Konservierungs- und/oder Deodorantwirkstoff, wobei das Polypeptid ein alpha-verknüpftes Homopolypeptid von Lysin mit einem Molekulargewicht von 15.000 bis 30.000 ist.

5. Zusammensetzung nach Anspruch 4, wobei das Polypeptid 0,05 bis 10% der Zusammensetzung ausmacht, die Trägerflüssigkeit 5 bis 99% der Zusammensetzung ausmacht und der Konservierungs- und/oder der Deodorantwirkstoff 0,01 bis 5% der Zusammensetzung ausmacht, wobei sich sämtliche Prozentangaben auf das Gewicht beziehen.

6. Zusammensetzung nach irgendeinem der Ansprüche 4 bis 5, umfassend einen Duftstoff.

7. Zusammensetzung nach einem der Ansprüche 4 bis 6, wobei die Trägerflüssigkeit Wasser umfasst.

8. Zusammensetzung nach einem der Ansprüche 4 bis 7, wobei die Trägerflüssigkeit Ethanol umfasst.

9. Produkt, umfassend eine Zusammensetzung nach einem der Ansprüche 4 bis 8 und einen Spender für die Zusammensetzung, wobei der Spender einen Behälter und ein Auftragungsmittel für die Zusammensetzung umfasst.

## Revendications

1. Utilisation cosmétique non thérapeutique d'un polypeptide comprenant de multiples résidus de lysine liés en alpha comme agent anti-transpirant, dans laquelle le polypeptide est un homopolypeptide de lysine lié en alpha ayant un poids moléculaire de 15 000 à 30 000.

2. Utilisation selon la revendication 1, dans laquelle le polypeptide est appliqué à partir d'une composition comprenant un fluide vecteur acceptable du point de vue cosmétique et un agent actif conservateur et/ou déodorant.

3. Utilisation selon l'une quelconque des revendications précédentes, dans laquelle le polypeptide est délivré à la surface de la peau à une concentration d'au moins 0,05 % en poids.

4. Composition anti-transpirante comprenant un polypeptide comprenant de multiples résidus de lysine liés en alpha; un fluide vecteur acceptable du point de vue cosmétique; et un agent conservateur et/ou déodorant actif, dans laquelle le polypeptide est un homopolypeptide de lysine lié en alpha ayant un poids moléculaire de 15 000 à 30 000.

5. Composition selon la revendication 4, dans laquelle le polypeptide représente de 0,05 à 10 % de la composition, le fluide vecteur représente de 5 à 99 % de la composition et l'agent actif conservateur et/ou déodorant représente de 0,01 à 5 % de la composition, tous les pourcentages étant en poids.

6. Composition selon l'une quelconque des revendications 4 à 5, comprenant un parfum.

7. Composition selon l'une quelconque des revendications 4 à 6, dans laquelle le fluide vecteur comprend de l'eau.

8. Composition selon l'une quelconque des revendications 4 à 7, dans laquelle le fluide vecteur comprend de l'éthanol.

9. Produit comprenant une composition selon l'une quelconque des revendications 4 à 8 et un distributeur pour ladite composition, le distributeur comprenant des moyens de réception et des moyens d'application pour la composition.
